# FASCICULE DE BREVET EUROPEEN

(11) **EP 1 176 946 B1**
(45) Date de publication et mention de la délivrance du brevet: **09.02.2011**
(21) Numéro de dépôt: 99939502.3
(22) Date de dépôt: 26.08.1999
(51) Int. Cl.: A61K 8/92, A61K 8/97, A61K 8/02, A61Q 19/08

(54) **COMPOSITION COSMETIQUE CONTENANT DES HUILES DE CYNARA CARDUNCULUS OU DE SILYBUM MARIANUM**
KOSMETISCHE ZUSAMMENSETZUNG ENTHALTEND ÖLE VON CYNARA CARDUNCULUS ODER SILYBUM MARIANUM
COSMETIC COMPOSITION CONTAINING CYNARA CARDUNCULUS OR SILYBUM MARIANUM OILS

(43) Date de publication de la demande: 06.02.2002
(73) Titulaire: Julia, Jean, 66550 Corneilla La Rivière (FR)
(72) Inventeur: Julia, Jean, 66550 Corneilla La Rivière (FR)
(74) Mandataire: Rhein, Alain
(86) Numéro de dépôt international: PCT/FR1999/002045
(87) Numéro de publication internationale: WO 2001/013879

(56) Documents cités:
- EP-A- 0 236 218
- EP-A- 0 519 727
- BE-A- 904 014
- FR-A- 2 716 371
- DATABASE WPI Section Ch, Week 199405 Derwent Publications Ltd., London, GB; Class B04, AN 1994-040014 XP002134775 & JP 05 345705 A (NIPPON SHINYAKU CO LTD), 27 décembre 1993 (1993-12-27)

## Description

La présente invention concerne une composition pour utilisation cosmétique et hygiène corporelle à base d'huile de Cynara Cardunculus ou de Silybum Marianum. Elle concerne également un procédé d'extraction de l'huile adapté à la fabrication d'une composition pour utilisation cosmétique et hygiène corporelle.

Certains sujets de la famille des chardons sont connus pour leur efficience à l'égard de certaines affections. C'est ainsi que Cnicus Benedictus exerce ses effets sur la digestion et la fièvre, la cardère (Dipsacus Silvestris) sur l'eczéma et l'acné, le Cynara Cardunculus et le Silybum Marianum sur le foie, les varices et les hémorroïdes. Egalement, il est connu du brevet JP5345705 des compositions cosmétiques contenant des extraits de Cynara Scolymus et Silybum Marianum, ainsi que l'emploi d'extraits de fruits de Silybum Marianum dans des compositions cosmétiques, comme mentionné dans la demande de brevet EP 0 236 218.

Il a été mis en évidence, ainsi qu'il est décrit dans le brevet français 2.716.371, que l'utilisation dans des produits cosmétiques de l'huile produite par pressurage à froid, sous haute pression, des graines de Cynara Cardunculus, donnait lieu à de bons résultats dans les domaines cutanés et capillaires.

Dans le prolongement du travail entrepris sur l'huile de Cynara Cardunculus, une étude systématique de cette huile a montré qu'il y avait possibilité d'améliorer de manière surprenante ses applications en l'associant à d'autres substances dont les composés agissent en synergie avec ceux de l'huile de Cynara Cardunculus. Une autre étude a montré que des résultats similaires pouvaient être obtenus avec une huile obtenue par pressurage à froid, sous haute pression, des graines de Silybum Marianum.

Selon la présente invention, est proposée une composition pour utilisation cosmétique et hygiène corporelle qui contient de l'huile produite par malaxage et pressurage à froid, sous haute pression, de graines de Cynara Cardunculus ou de Silybum Marianum non décortiquées, ladite huile contenant de la silymarine favorisant l'absorption cutanée de la composition, et d'autre part, un extrait de feuilles de Cynara Cardunculus, plante de la famille des chardons, ledit extrait contenant de la cynarine et de l'acide chlorogénique.

L'huile produite par malaxage et pressurage à froid, sous haute pression, des graines de Cynara Cardunculus ou de Silybum Marianum contient de la silymarine directement bio-disponible à dose importante et constante (276 mg par litre, soit 300 mg par kilogramme pour l'huile de Cynara Cardunculus). Elle contient également une quantité importante d'acide linoleïque et d'acide oleïque, des acides aminés, notamment proline et glycine, des phytosterols contenus dans les insaponifiables, de la vitamine E et du béta-carotène biodisponibles.

Un extrait de plante de la famille des chardons, comprenant notamment le cynara cardunculus, le silybum marianum, le dipsacus silvestris, le cnicus benedictus et le cynara scolymus, contient notamment des polyphénols dont des dérivés caféiques, notamment cynarine et acide chlorogénique, et des flavonoïdes.

La silymarine favorise l'absorption cutanée de la composition. En outre, la silymarine agissant en synergie avec la cynarine a un effet anti-radicalaire important. L'effet anti-oxydant et de piège des radicaux libre est renforcé par la présence dans la composition d'autres antioxydants tels que la vitamine E et le béta-carotène. Ces constituants permettent en outre à des acides aminés tels que la proline et la glycine de régénérer le collagène dont ils sont les composants principaux. Or, le collagène est avec l'élastine un des principaux éléments permettant une bonne tenue des tissus épidermiques.

En outre, l'acide chlorogénique est un veinotonique qui va stimuler la circulation dans les capillaires de surface, ce qui va favoriser l'élimination des toxines. Cette action va coopérer avec l'action des flavonoïdes connus pour leur action entraînant l'amélioration des mécanismes d'élimination des lipides. La silymarine du fait de son affinité membranaire, diminue le passage membranaire et entraîne la baisse de la pénétration des toxiques.

Selon un autre aspect de la présente invention, la composition se présente sous forme de lait, gel, crème, pommade, baume, émulsion, shampoing ou masque. Elle contient entre 0,5% et 98% d'huile, selon l'effet recherché. Avantageusement, elle contient entre 5% et 25% d'huile, notamment pour les laits et crèmes. Selon un mode de réalisation actuellement préféré pour une crème ou un lait, elle contient environ 10% d'huile.

De la quantité d'huile présente dans la composition dépend directement la teneur de la composition en silymarine, vu la stabilité de la teneur en silymarine de l'huile produite par malaxage et pressurage à froid, sous haute pression, des graines de Cynara Cardunculus ou de Silybum Marianum.

Selon la présente invention, l'extrait en question est un extrait de feuilles de Cynara Cardunculus, plante de la famille des chardons.

Les feuilles sont en effet idéales pour l'extraction des principes actifs, notamment la cynarine et les flavonoïdes. La feuille de Cynara Cardunculus contient ces principes actifs en grande quantité.

Selon un autre aspect de la présente invention, la composition contient entre 0,1 et 50% de l'extrait de feuilles de cynara carolunculus en fonction de la composition de cet extrait. Avantageusement, elle contient entre 0,1 et 50% dudit extrait en fonction de la teneur de l'extrait en polyphénols tels que dérivés caféiques et flavonoïdes. Avantageusement, notamment lorsque la composition contient environ 10% d'huile, elle contient entre 0,1 et 10% de l'extrait

La teneur en principes actifs de l'extrait de plante de la famille des chardons dépend en effet de la période de récolte de la plante ainsi que de la technique d'extraction. Une étude a montré que les concentrations de dérivés caféiques totaux et de flavonoïdes totaux sont respectivement 1,65 fois et 3,5 fois plus importants au printemps qu'en automne dans des extraits d'artichauts obtenus selon le même protocole. De même, une étude a montré qu'une technique d'extraction en milieu aqueux à chaud est la mieux adaptée lorsque l'on souhaite obtenir un extrait riche en acide chlorogénique. Par contre, le traitement des feuilles de Cynara Cardunculus par une solution hydro-alcoolique à 45/55 % vol/vol semble mieux convenir à l'extraction de la cynarine.

Sont décrits dans ce qui suit des modes de réalisation préférés de la présente invention.

### 1.Obtention d'une huile de graines de Cynara Cardunculus.

Des Cynara Cardunculus sont cultivées. La plante se cultive comme un tournesol, la culture est totalement mécanisée. La récolte se fait à la moissonneuse batteuse classique axiale pour ne pas endommager les graines. Les rendements en graines oléagineuse sont de l'ordre de 1,5 à 2 tonnes par hectare en culture normalement menée. Ces graines ont une teneur en huile de 16 à 18% pour le premier cycle de culture et de 22 à 26% pour les suivants. Ceci donne lieu, dans le cas d'une extraction par pression à froid, à un pourcentage de 12 à 14% d'huile vierge lors du premier cycle, et de 15 à 18% pour les suivants.

L'extraction d'huile se fait exclusivement par première pression sans chauffage des graines non décortiquées. La température ne dépasse pas celle due aux frottements des pièces métalliques, dont le maximum est de l'ordre de 40° à 50°. Les protocoles de trituration des graines et extraction de l'huile sont ceux décrits dans le cahier des charges de l'agriculture biologique.

L'huile ainsi obtenue contient notamment les éléments suivants.

### Acides Gras.

| | |
|---|---|
| Acide Myristique | 0,12 % |
| Acide Palmitique | 11,12 % |
| Acide Stéarique | 2,75 % |
| Acide Oléïque | 21,15 % |
| Acide Linoleïque | 63,10 % |
| Acide Arachidique | 0,30% |
| Acide γ-Linolénique | 0,05 % |
| Acide α-Linolénique | 0,33 % |

### Fractions d'esters.

| | |
|---|---|
| β-Sistostérol | 43,1 % |
| δ-7 Stigmastereol | 26,7% |
| Stigmastereol | 13,1 % |

### Acides Aminés.

### Acides Aminés pour 100 grammes.

| | |
|---|---|
| Acide aspartique | 1800 mg |
| Thréonine | 790 mg |
| Sérine | 880 mg |
| Acide Glutamique | 2380 mg |
| Proline | 3370 mg |
| Glycine | 6610 mg |
| Alanine | 2760 mg |
| Cystine totale | 190 mg |
| Valine | 760 mg |
| Methionine | 180 mg |
| Isoleucine | 400 mg |
| Leucine | 850 mg |
| Tyrosine | 150 mg |
| Phényllalanine | 510 mg |
| Lysine | 910 mg |
| Histidine | 170 mg |
| Arginine | 1980 mg |
| Tryptophane | 50 mg |

### Autres composants.

| | |
|---|---|
| Silymarine | 276 mg/l |
| β-Carotène | 4 mg/l |
| Vitamine E | 270 mg/l |

En ce qui concerne les acides gras essentiels, l'huile contient une grande quantité d'acides linoleïque et oleïque. Elle contient 65% d'acides gras poly insaturés et 22% d'acides gras mono insaturés. Elle est idéalement dosée pour avoir un effet majeur dans un rôle de précurseur des prostaglandines.

Les acides gras essentiels contenus dans l'huile interviennent également au niveau de la structure des membranes cellulaires.

En ce qui concerne les acides aminés, tous les acides aminés essentiels sont présents dans l'huile de Cynara Cardunculus. Ils ont notamment un rôle de vecteurs, de transmetteurs. Ils interviennent également dans le domaine hormonal, au niveau de la cellule, au niveau immunitaire, et sont des détoxiquants.

En ce qui concerne les esters contenus dans les insaponifiables, les phytostérols contenus dans l'huile de Cynara Cardunculus sont correctement dosés et répartis au sein des insaponifiables. On sait notamment qu'ils contribuent à la lutte contre le dépôt de cholestérol en particulier dans le système cardio-vasculaire. Des recherches ont montré que l'absorption de phytostérols réduisait de manière notable le taux de cholestérol sanguin. Ils amènent également une protection accrue de la cellule. De plus, ces substances ont un effet important en tant que substituts aux oestrogènes naturels chez la femme ménopausée ou pré-ménopausée.

En ce qui concerne la vitamine E, elle est contenue en grande quantité dans l'huile de Cynara Cardunculus sous forme naturelle RRR-d-alpha tocophérol. Cette forme naturelle possède une bio-disponibilité deux fois supérieure à celle de la forme synthétique de la vitamine E. La vitamine E se comporte comme un anti-oxydant, protégeant de l'oxydation les lipides circulant et les lipides des membranes cellulaires.

L'huile contient également du bêta-carotène qui est notamment le précurseur de la vitamine A dans l'organisme.

En ce qui concerne la silymarine contenue dans l'huile de Cynara Cardunculus, elle est directement bio-disponible, ce qui n'est pas le cas, par exemple, de la silymarine extraite chimiquement de la balle de l'akène du chardon marie.

Selon des études menées sur la silymarine, cette dernière a une compatibilité membranaire avec la cellule. Son action se traduit notamment par une action protectrice vis à vis des lésions produites par les toxiques, du fait de l'affinité membranaire diminuant la perméabilité de la membrane, et par une action régénératrice, du fait de l'augmentation de la multiplication des cellules qu'elle entraîne. Elle favorise l'augmentation de la synthèse protéique des mitochondries épithéliales induisant une régénération accélérée des tissus épithéliaux.

### 2. Obtention d'un extrait de feuilles de Cynara Cardunculus.

L'extrait est obtenu à partir de feuilles entières congelées de Cynara Cardunculus. Un lot de feuilles a été récolté au printemps, l'autre en automne. Trois procédés d'extraction ont été utilisés.

### Extrait 1.

Un extrait aqueux a été obtenu à partir de 4,8 kg de feuilles d'automne congelées coupées et 7,2 kg d'eau. L'ensemble a été porté à ébullition pendant deux heures en limitant l'évaporation. La partie solide est ensuite récupérée à l'aide d'un tamis grossier et la partie liquide, ou « jus » est réservée. La partie solide est comprimée à l'aide d'un pressoir à vis, et le liquide obtenu est ajouté au « jus » réservé et le tout est refroidi à 4° C pendant 24 heures. Le tout, après ajout de conservateur, est ensuite filtré.

### Extrait 2.

Un extrait glycolique a été obtenu par macération à partir de 20 g de feuilles d'automne congelées coupées, 95 g de propylène glycol, 50 g d'eau et 2g de conservateur. La macération est menée à température ambiante dans un bêcher en verre à l'abri de la lumière. La plante est agitée tous les 3 ou 4 jours à l'aide d'un baton téflonné. La macération dure 3 semaines, après quoi la solution est recueillie par tamisage puis compression dans un linge, puis filtrage au papier filtre. Après estimation de la teneur en propylène glycol par réfractométrie, on rajoute du propylène glycol afin d'obtenir une solution contenant autant d'eau que de propylène glycol.

### Extrait 3.

Un extrait aqueux est obtenu dans les mêmes conditions que pour l'extrait 1, mais à partir de feuilles récoltées au printemps, juste avant la floraison (apparition du bouton).

Un dosage effectué sur les trois extraits a donné les résultats suivants.

| **Extrait analysé** | **Dérivés caféiques (g/l)** | **Flavonoïdes (g/l)** |
|---|---|---|
| *Extrait 1* | 2,59 | 0,147 |
| *Extrait 2* | 0,654 | 0,102 |
| *Extrait 3* | 4,29 | 0,510 |

Il est à noter que, vu le procédé de dosage utilisé dans cette étude, les concentrations en dérivés caféiques peuvent être surévaluées. Ces concentrations permettent néanmoins de bien constater les variations en fonction de la période de récolte des plantes et du procédé d'extraction.

En ce qui concerne la teneur plus particulière en acide chlorogénique et en cynarine, un dosage a donné les résultats suivants.

| **Extrait analysé** | **Acide chlorogénique (g/l)** | **Cynarine (g/l)** |
|---|---|---|
| *Extrait 1* | 0,353 | 0,172 |
| *Extrait 2* | 0,061 | 0,126 |
| *Extrait 3* | 0,557 | 0,868 |

### 3.Obtention d'une composition pour utilisation cosmétique et hygiène corporelle.

L'on donne ici plusieurs exemples de compositions cosmétiques et d'hygiène corporelle utilisant un mélange d'huile de graines de Cynara Cardunculus telle qu'obtenue de la manière décrite en (1) et d'un extrait aqueux de feuilles récoltées au printemps de Cynara Cardunculus (extrait 3) tel qu'obtenu de la manière décrite en (2).

### 1.Crème de jour.

Cette composition contient :
- 10% d'huile ;
- 2% d'extrait de feuilles ;
- filtre solaire ;
- céramides végétaux ;
- facteur hydratant.

Son application permet d'hydrater et de nourrir en profondeur la peau. La circulation dans les capillaires de surface est stimulée. En surface, un film protecteur est formé, lissant et adoucissant l'épiderme. En profondeur, la composition active la régénération de la peau et la réparation des désordres intracellulaires tout en facilitant la circulation, donc, l'élimination des toxines.

### 2.Soin de nuit.

Cette composition contient :
- 10% d'huile ;
- 2% d'extrait de feuilles;
- céramides végétaux ;
- texture nutritive

### 3.Soin anti age.

Cette composition contient :
- 10% d'huile ;
- 5% d'extrait de feuilles;
- facteur A. R. L. ;
- extrait de ginseng;
- allantoine;
- céramides végétaux.

L'association des flavonoïdes de l'huile (silymarine), ceux contenus dans le complexe A.R.L. et ceux contenus dans l'extrait de feuilles opère une action régénératrice intense.

L'extrait de ginseng renforce la tonification, les céramides végétaux renforcent la protection des couches superficielles de l'épiderme, l'allantoine calme les irritations cutanées et favorise la multiplication cellulaire.

### 4.Masque crème.

Cette composition contient :
- 5% d'huile ;
- 5% d'extrait de feuilles;
- extrait de milleperthuis.

Elle combine tout à la fois des fonctions régénérante, désincrustante, veinotonique, apaisante et lissante.

### 5. Lait corporel.

Cette composition contient :
- 18% d'huile;
- 5% d'extrait de feuilles;
- agents hydratants ;
- céramides végétaux.

Il s'agit d'un lait corporel particulièrement hydratant. Sa haute teneur en silymarine (10 mg par flacon de 200 mg) permet une absorption cutanée maximale. Il va permettre à la peau de lutter contre les radicaux libres et les toxines issues d'agressions externes. L'extrait de feuilles renforce le pouvoir anti-oxydant, notamment du fait de la présence de cynarine, et apporte un effet tonifiant et veinotonique avec l'acide chlorogénique. Les flavonoïdes absorbés ont un effet détoxiquant qui permet de lutter efficacement contre les désordres intra-cellulaires.

### 6. Lait démaquillant

Cette composition contient :
- 10% d'huile ;
- 2% d'extrait de feuilles;
- agents hydratants ;
- céramides végétaux.

Tous les composants des compositions qui viennent d'être décrites ont en commun des champs d'action sur l'organisme humain. Leurs actions s'opèrent en synergie et leurs effets s'en trouvent accrus par rapport à ce qu'ils seraient individuellement.

## Revendications

1. Composition pour utilisation cosmétique et hygiène corporelle, **caractérisée en ce qu'**elle contient, d'une part, de l'huile produite par malaxage et pressurage à froid, sous haute pression, de graines de Cynara Cardunculus ou de Silybum Marianum non décortiquées, ladite huile contenant de la silymarine favorisant l'absorption cutanée de la composition, et d'autre part, un extrait de feuilles de Cynara Cardunculus, plante de la famille des chardons, ledit extrait contenant de la cynarine et de l'acide chlorogénique.

2. Composition pour utilisation cosmétique et hygiène corporelle selon la revendication 1 **caractérisée en ce que**, se présentant sous forme de lait, gel, crème, pommade, baume, émulsion, shampoing ou masque, elle contient entre 0, 1 % et 98% de ladite huile.

3. Composition pour utilisation cosmétique et hygiène corporelle selon la revendication 1 **caractérisée en ce qu'**elle contient entre 5% et 25% de ladite huile.

4. Composition pour utilisation cosmétique et hygiène corporelle selon la revendication 1 **caractérisée en ce qu'**elle contient environ 10% de ladite huile.

5. Composition pour utilisation cosmétique et hygiène corporelle selon l'une quelconque des revendications précédentes, **caractérisée en ce qu'**elle contient entre 0,1 et 50% dudit extrait de feuilles.

## Claims

1. Composition for cosmetic use and body hygiene, wherein it contains, on the one hand, oil produced by cold mixing and pressing, under high pressure, of non-decorticated Cynara Cardunculus or Silybum Marianum grains, said oil containing silymarine promoting cutaneous absorption of the composition and, on the other hand, an extract of Cynara Cardunculus leaves, plant of the cardoon species, said extract containing cynarine and chlorogenic acid.

2. Composition for cosmetic use and body hygiene according to claim 1, wherein, being in the form of milk, gel, cream, ointment, balsam, emulsion, shampoo or a mask, it contains from 0.1% to 98% of said oil.

3. Composition for cosmetic use and body hygiene according to claim 1, wherein it contains from 5% to 25% of said oil.

4. Composition for cosmetic use and body hygiene according to claim 1, wherein it contains approximately 10% of said oil.

5. Composition for cosmetic use and body hygiene according to any of the preceding claims, wherein it contains from 0.1 to 50% of said leaf extract.

## Patentansprüche

1. Zusammensetzung für kosmetische Verwendung und Körperhygiene, **dadurch gekennzeichnet, dass** sie zum einen durch Kaltkneten und -auspressen unter hohem Druck von nichgeschälten Cynara Cardunculus- oder Silybum Marianum-Körnern produziertes Öl, wobei das besagte Öl Silymarin enthält, das die Aufnahme der Zusammensetzung durch die Haut befördert, und zum anderen einen Blätterextrakt von Cynara Cardunculus, Pflanze der Distelfamilie, enthält, wobei der besagte Extrakt Cynarin und Chlorogensäure enthält.

2. Zusammensetzung für kosmetische Verwendung und Körperhygiene nach Anspruch 1, **dadurch gekennzeichnet, dass** sie, wenn sie in der Form von Milch, Gel, Creme, Salbe, Balsam, Emulsion, Shampoo oder einer Maske ist, von 0,1% bis zu 98% des besagten Öls enthält.

3. Zusammensetzung für kosmetische Verwendung und Körperhygiene nach Anspruch 1, **dadurch gekennzeichnet, dass** sie von 5% bis zu 25% des besagten Öls enthält.

4. Zusammensetzung für kosmetische Verwendung und Körperhygiene nach Anspruch 1, **dadurch gekennzeichnet, dass** sie ungefähr 10% des besagten Öls enthält.

5. Zusammensetzung für kosmetische Verwendung und Körperhygiene nach irgendeinem der vorgehenden Ansprüche, **dadurch gekennzeichnet, dass** sie von 0,1 bis zu 50% des besagten Blätterextraktes enthält.
